(19) Europäisches Patentamt European Patent Office Office européen des brevets

(11) **EP 3 858 408 B1**

(12) **EUROPEAN PATENT SPECIFICATION**

(45) Date of publication and mention
of the grant of the patent:
**19.03.2025 Bulletin 2025/12**

(21) Application number: **19867758.5**

(22) Date of filing: **24.09.2019**

(51) International Patent Classification (IPC):
**A61M 16/00** (2006.01)   **A61B 5/08** (2006.01)
**A61M 16/10** (2006.01)   **A61B 5/087** (2006.01)
**A61M 16/16** (2006.01)   **B01D 53/047** (2006.01)

(52) Cooperative Patent Classification (CPC):
**A61M 16/101; A61B 5/087; A61M 16/024;**
**B01D 53/047;** A61B 2505/07; A61M 16/0063;
A61M 16/026; A61M 16/0672; A61M 16/16;
A61M 16/202; A61M 2016/0021; A61M 2016/0027;
A61M 2016/0039; A61M 2202/0208;
A61M 2205/3331;                           (Cont.)

(86) International application number:
**PCT/JP2019/037391**

(87) International publication number:
**WO 2020/067067 (02.04.2020 Gazette 2020/14)**

(54) **BREATHING INFORMATION ACQUISITION DEVICE**

VORRICHTUNG ZUR ERFASSUNG VON ATMUNGSINFORMATION

DISPOSITIF D'ACQUISITION D'INFORMATIONS DE RESPIRATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **25.09.2018   JP 2018178914**

(43) Date of publication of application:
**04.08.2021   Bulletin 2021/31**

(73) Proprietor: **Teijin Pharma Limited**
**Tokyo 100-0013 (JP)**

(72) Inventors:
• **SUGANO, Masato**
**Tokyo 100-0013 (JP)**

• **YOSHIZAWA, Akira**
**Tokyo 100-0013 (JP)**

(74) Representative: **Hoffmann Eitle**
**Patent- und Rechtsanwälte PartmbB**
**Arabellastraße 30**
**81925 München (DE)**

(56) References cited:
WO-A1-2014/059405   WO-A1-2018/180392
FR-A1- 2 930 165   JP-A- 2014 068 782
JP-A- 2015 085 191   JP-A- 2016 220 924
JP-A- H06 190 045   JP-A- H06 190 045

EP 3 858 408 B1

**(Cont. next page)**

(52) Cooperative Patent Classification (CPC): (Cont.)
A61M 2230/40; B01D 2256/12; B01D 2259/40007;
B01D 2259/40009; B01D 2259/4533

**Description**

FIELD

The present disclosure relates to a respiratory information acquisition device used in a PSA-type oxygen concentrator.

BACKGROUND

Oxygen therapy has traditionally been used as one of the treatments for patients with respiratory diseases such as asthma and obstructive chronic lung disease. This is a therapy in which oxygen gas or concentrated oxygen gas is inhaled by a patient. In recent years, home oxygen therapy (HOT) in which oxygen is inhaled at home or at facilities for the purpose of improving patient QOL has become mainstream, and an oxygen concentrator is mainly used as the oxygen supply source.

An oxygen concentrator is a device which concentrates and discharges approximately 21% of the oxygen present in air. In many oxygen concentrators, a pressure swing adsorption type (hereinafter, PSA-type) is generally used (hereinafter, "PSA-type oxygen concentrator" is simply referred to as "oxygen concentrator").

In PSA-type oxygen concentrators, an adsorption step of drawing air into adsorption columns in which an adsorbent for selectively adsorbing nitrogen gas is filled and pressurizing the adsorption columns to adsorb nitrogen gas onto the adsorbent and a desorption step of reducing the pressure in the adsorption columns and discharging the adsorbed nitrogen gas out of the system are repeated. By repeating the adsorption step and the desorption step, concentrated oxygen gas is generated, and the oxygen concentrator can continuously provide high-concentration oxygen gas to the patient. Furthermore, a method of reducing the pressure in the adsorption columns to atmospheric pressure or less in the desorption step is sometimes referred to as VPSA or VSA rather than PSA, but the basic principles are the same as those of PSA, and thus, will be uniformly referred to hereinafter as PSA.

Chronic Obstructive Pulmonary Disease (COPD) is the primary disease of patients who receive home oxygen therapy. COPD is an irreversible disorder which presents with symptoms such as coughing and sputum, and dyspnea during exertion due to bronchial stenosis and destruction of the alveolar walls.

Worsening symptoms of COPD may result in shortness of breath and increased respiratory rate, and symptoms may worsen until "changes in treatment or conditions requiring additional therapy during the stable phase", referred to as acute exacerbation of COPD. When an acute exacerbation of COPD occurs, the patient is often hospitalized and may face respiratory failure or death. Even if the patient can be discharged from the hospital, the symptoms during the stable phase are worse than before hospitalization, and it is not uncommon for the patient to be repeatedly hospitalized and discharged.

In COPD, it is very important that signs or early symptoms acute exacerbation of a patient be detected as early as possible, and early treatment be provided to the patient prior to symptom deterioration to the extent that hospital admission is required. In the home oxygen therapy, the respiratory information of the patient becomes a very useful information source for understanding the state of the disease of the patient.

It is very important that the patient breath concentrated oxygen gas in accordance with the prescribed flow rate diagnosed by the physician. However, it is often the case that oxygen cannot be properly breathed due to displacement of the cannula during sleep or dislodgement of the tube during work, and the patient may intentionally remove the cannula for some reason even when the oxygen concentrator is in operation. In any case, a situation in which the patient is unable to breath the oxygen even when the oxygen concentrator is operating may lead to deteriorate of the condition of the patient.

Thus, if the oxygen concentrator can acquire patient respiratory information inside the oxygen concentrator during operation, the oxygen concentrator can acquire the respiratory information of the patient without changes in usability as compared with conventional general oxygen concentrator. Further, an oxygen concentrator which can acquire patient respiratory information is very useful because it can confirm that the oxygen concentrator and the patient are connected by an oxygen supply tube and the actual usage of the patient.

[CITATION LIST]

[PATENT LITERATURE]

[PTL 1] Japanese Unexamined Patent Publication (Kokai) No. 6-190045
[PTL 2] Japanese Unexamined Patent Publication (Kokai) No. 2001-286566
[PTL 3] Japanese Unexamined Patent Publication (Kokai) No. 7-96035
[PTL 4] Japanese Unexamined Patent Publication (Kokai) No. 2015-85191

[PTL 5] Japanese Unexamined PCT Publication (Kohyo) No. 2011-518016
WO 2014/059405 AI relates to an oxygen concentrator system and method of delivering oxygen enriched gas to a user. The oxygen concentrator system includes one or more components that improve the efficiency of oxygen enriched gas delivery during operation of the oxygen concentrator system.

SUMMARY

The invention is defined by the appended claims.

As a method of acquiring respiratory information of a patient during operation of an oxygen concentrator, there is a method of measuring a patient respiration pressure during oxygen inhalation by attaching a fine differential pressure sensor for respiration measurement between an oxygen concentrator and a cannula worn by a patient, as shown in Patent Literature 1 and 2. However, as a result of rigorous investigation conducted by the present inventors, it has been clarified that the pressure fluctuations added to the differential pressure measurement system includes pressure fluctuations due to the pressurization and depressurization at the time of generating the concentrated oxygen gas. As described above, the oxygen concentrator uses a PSA-type operation method, and the pressure of the concentrated oxygen gas drawn from the adsorption columns is affected by the pressure fluctuations caused by the pressurization and depressurization of the adsorption columns, and generally fluctuates greatly. In many oxygen concentrators, a buffer tank is provided on an oxygen outlet side of an adsorption columns to suppress pressure fluctuations, and a pressure regulating valve is provided on an outlet side of the buffer tank, so that the pressure of the concentrated oxygen gas supplied to a patient is maintained substantially constant. In practice, slight fluctuations remain in the pressure of the concentrated oxygen gas, and such slight fluctuations hinder the acquisition of respiratory information. In other words, in an environment in which patient respiration pressure is simply measured by a fine differential pressure sensor, the patient respiration pressure is detected based on pressure fluctuations which are affected by PSA operation, and thus, the patient respiratory information cannot be accurately acquired. In particular, with the addition of an extension tube or at a flow rate of 3.0 L/min or higher, it becomes impossible to obtain patient respiratory information.

In addition, in Patent Literature 3 and 4, there is provided a method in which patient respiration pressure during oxygen inhalation is measured by a fine differential pressure sensor, and the obtained respiration pressure data is then soft-processed to obtain patient respiratory information. However, in these prior art technologies, since the pressure fluctuations associated with PSA operation which are superimposed on the patient respiration pressure are not taken into consideration, the respiratory information of the patient cannot be accurately acquired.

Patent Literature 5 describes a method for obtaining respiratory information by analyzing obtained respiration pressure data by frequency analysis. However, in the frequency analysis, there is a drawback in that it is difficult to separate respiration components and pressure fluctuation components accompanying PSA operation, thus resulting in a poor accuracy

In consideration of the foregoing, in order to acquire patient respiratory information during oxygen concentrator operation, it is necessary to separate patient respiration components and variable pressure components associated with PSA operation from the measured data of the patient respiration pressure.

The respiratory information acquisition device aims to acquire patient respiratory information by separating patient respiration components from variable pressure components associated with PSA operation during oxygen concentrator operation.

An aspect of an embodiment provides a respiratory information acquisition device which acquires respiratory information of a patient and which is used in an oxygen concentrator comprising an oxygen generation unit which has an adsorption columns having therein an adsorbent for preferentially adsorbing at least nitrogen gas in air and which uses a pressure swing adsorption method to concentrate oxygen in air by periodically repeating pressurization and depressurization in the adsorption columns, and an oxygen supply unit which supplies concentrated oxygen gas which has been concentrated by the oxygen generation unit to the patient, which is a user, wherein the oxygen supply unit has at least a conduit for delivering the concentrated oxygen gas to the patient, the respiratory information acquisition device has a pressure detection unit for detecting pressure in the conduit and/or a flow rate detection unit for detecting a gas flow rate in the conduit, as well as a calculation unit for extracting oxygen respiratory information from detected pressure data and/or flow rate data, and the calculation unit estimates a fluctuation component which does not depend on the respiration of the patient based on the detected pressure data and/or flow rate data and information related to an operation state of the oxygen concentrator obtained from the oxygen concentrator, and extracts respiratory information by removing the estimated fluctuation component from the detected pressure data and/or flow rate data.

The respiratory information acquisition device according to an aspect of an embodiment is characterized in that the calculation unit estimates a fluctuation value generated in accordance with operation of the oxygen generation unit among the detected pressure data and/or flow rate data as the fluctuation component.

The respiratory information acquisition device according to an aspect of an embodiment is characterized in that the calculation unit uses a fluctuation value of the pressure and/or the gas flow rate in the conduit associated with the pressurization and/or depressurization in the adsorption columns as the fluctuation value.

The respiratory information acquisition device according to an aspect of an embodiment is characterized in that the calculation unit acquires switching period information related to a switching period at which the pressurization and depressurization in the adsorption columns is switched from the oxygen concentrator as the information related to the operation state of the oxygen concentrator, and estimates the fluctuation value based on the detected pressure data and/or flow rate data and the switching period information.

The respiratory information acquisition device according to an aspect of an embodiment is characterized in that the calculation unit estimates the fluctuation component of the pressure and/or gas flow rate generated in the same interval as the switching period included in the detected pressure data and/or flow rate data as the fluctuation value of the pressure and/or gas flow rate associated with the pressurization and/or depressurization in the adsorption columns.

The respiratory information acquisition device according to an aspect of an embodiment is characterized in that the oxygen generation unit switches between pressurization and depressurization of the adsorption columns at a constant interval T for at least a part of a total operation time, and the calculation unit extracts the respiratory information by calculating an averaging processed value X(t) using at least a part of the pressure or gas flow rate value Y(t) in the conduit at a certain time (t) and the values Y(t-T), Y(t-2T), ..., at the time retroactive by an integral multiple of T from t, among the detected pressure data and/or flow rate data, as the fluctuation value, and calculating the difference between Y(t) and X(t).

The respiratory information acquisition device according to an aspect of an embodiment is characterized in that the value X(t) is calculated by the formula represented by:

[Math 1]

$$X(t) = \frac{\sum_{i=0}^{n} a_i Y(t - iT)}{\sum_{i=0}^{n} a_i}$$

where $a_i$ is an arbitrary real number and n is an arbitrary natural number.

The respiratory information acquisition device according to an aspect of an embodiment is characterized in that exponential smoothing averaging, moving averaging, or weighted moving averaging is performed as the averaging processing.

The respiratory information acquisition device according to an aspect of an embodiment is characterized in that the difference between an actual value of the pressure and/or gas flow rate in the conduit at t and a time average value of the pressure in the conduit and/or the flow rate in the conduit at t over a time equal to or greater than a respiration interval is used as the Y(t).

The respiratory information acquisition device according to an aspect of an embodiment is characterized in that the pressure detection unit is a differential pressure sensor, the device further comprises a pressure smoothing unit, and a differential pressure between the pressure in the conduit at t and the pressure branched from the conduit and passed through the pressure smoothing unit is measured as Y(t).

The respiratory information acquisition device according to an aspect of an embodiment is characterized in that the pressure smoothing unit is constituted by a volume unit connected to the differential pressure sensor and an orifice for connection between the conduit and the volume unit.

According to the present embodiment, the respiratory information acquisition device can acquire the respiratory information of the patient by separating patient respiration components and variable pressure components associated with PSA operation during oxygen concentrator operation.

The objects and effects of the present invention will be recognized and obtained specifically by using the components and combinations indicated in the claims. Both the general description described above and the detailed description below are exemplary and descriptive and do not limit the invention described in the claims.

BRIEF DESCRIPTION OF DRAWINGS

FIG. 1 is a view showing an example of the structure of an oxygen concentrator according to embodiment 1.

FIG. 2 is a view showing an example of the structure of a respiratory information acquisition device according to embodiment 1.

FIG. 3 is a view showing pressure data including patient respiratory information under the conditions of a continuous flow of 1 LPM and a 10 m extension tube.

FIG. 4 is a view showing PSA pressure data extracted by a calculated unit under the conditions of a continuous flow of 1 LPM and a 10 m extension tube.

FIG. 5 is a view showing patient respiratory information after difference processing under the conditions of a continuous flow of 1 LPM and a 10 m extension tube.

FIG. 6 is a view showing pressure data including patient respiratory information under the conditions of a continuous flow of 3 LPM and a 10 m extension tube.

FIG. 7 is a view showing pressure data extracted by a calculation unit under the conditions of a continuous flow of 3 LPM and a 10 m extension tube.

FIG. 8 is a view showing patient respiratory information after difference processing under the conditions of a continuous flow of 3 LPM and a 10 m extension tube.

FIG. 9 is a conceptual diagram showing the principle of separating patient respiratory information and PSA pressure from pressure data including pressure fluctuations due to patient respiration and PSA pressure.

FIG. 10 is a flowchart showing an example of processing according to embodiment 1.

FIG. 11 is a flowchart showing an example of processing according to embodiment 2.

DESCRIPTION OF EMBODIMENTS

The respiratory information acquisition device according to aspects of the present disclosure with be described below with reference to the drawings.

The embodiments of the present technology will be described below with reference to the drawings.

[Hardware Configuration of Embodiment]

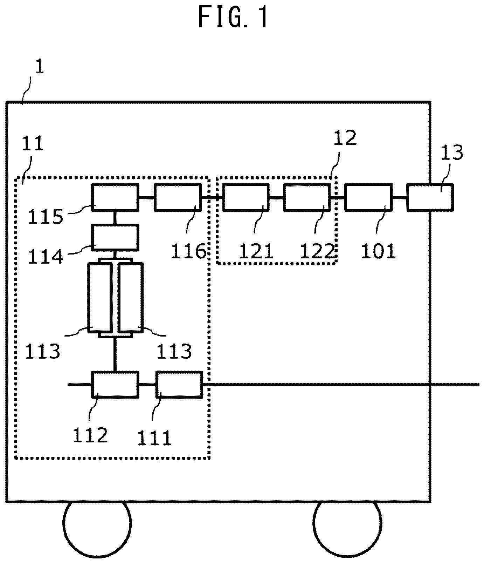

The structure of a PSA-type oxygen concentrator according to the present embodiment will be described using FIG. 1.

The PSA-type oxygen concentrator 1 comprises an oxygen generation unit 11 which draws in air from the outside of the PSA-type oxygen concentrator 1 and generates concentrated oxygen gas.

Air drawn into the oxygen generation unit 11 from outside the oxygen device is compressed by a compressor 111 and sent to an adsorption columns 113 via a first switching valve 112. The first switching valve 112 connects the compressor 111 with one of a plurality of adsorption columnss 113 to feed compressed air into the adsorption columns 113, and also opens the other adsorption columns(s) to the atmosphere. The adsorption columnss 113 are each filled with an adsorbent which selectively adsorbs nitrogen gas. The compressed air that has passed through the adsorption columns 113 has a reduced nitrogen gas concentration and becomes concentrated oxygen gas. The concentrated oxygen gas is stored in a concentrated oxygen buffer tank 115 via a second switching valve 114. The second switching valve 114 connects or disconnects one of the plurality of adsorption columnss 113 to or from the concentrated oxygen buffer tank 115.

The oxygen generation unit 11 connects the compressor 111 and one of the plurality of adsorption columnss 113 by the first switching valve 112, and connects the adsorption columns 113 connected with the compressor 111 by the second switching valve 114 and the concentrated oxygen buffer tank 115. Accordingly, the compressor 111, one of the plurality of adsorption columnss 113, and the concentrated oxygen buffer tank 115 are connected with each other, and the generated concentrated oxygen gas is supplied to the concentrated oxygen buffer tank 115. Conversely, the adsorption columnss 113 which are not connected with the compressor 111 are open to the atmosphere via the first switching valve 112 in a state shut off from the concentrated oxygen buffer tank 115 by the second switching valve 114. Thus, the pressure of the interior of the adsorption columns 113 is reduced, and the nitrogen gas adsorbed on the adsorbent is released to the outside of the oxygen concentrator.

The opening and closing of the first switching valve 112 and the second switching valve 114 is controlled by, for example, a calculation unit (not illustrated) of a respiratory information acquisition device. Thus, the calculation unit can obtain a period for switching between the pressurization and depressurization of the adsorption columns 113. Note that the respiratory information acquisition device may be installed inside the oxygen concentrator 1, or may be installed outside the oxygen concentrator 1 separately from the oxygen concentrator 1.

Furthermore, as another example, the oxygen concentrator 1 may have an oxygen concentration control unit which controls the oxygen concentration process including an opening/closing process of the first and second switching valves. The calculation unit can acquire the period at which the pressurization and depressurization in the adsorption columns 113 are switched from the oxygen concentration control unit.

Though the present embodiment is a basic configuration of oxygen concentration by pressure fluctuation adsorption, as another example, the present embodiment may have an additional step such as a pressure equalization step in which two or more adsorption columnss of the plurality of adsorption columnss 113 are connected to equalize the pressure of each of the adsorption columnss, or a purge step wherein a part of the generated concentrated oxygen gas is refluxed to one of the plurality of adsorption columnss 113.

Conventionally, after compressed air is sent to the adsorption columns 113, the latter is isolated from the compressor 111 by the first switching valve 112 and is opened to the atmosphere. Conversely, the adsorption columns 113 which has been opened to the atmosphere is connected to the compressor 111 by the first switching valve 112, and the oxygen compression process begins. Thus, the plurality of adsorption columnss 113 alternately repeat compression and release to the atmosphere by the first switching valve 112, whereby concentrated oxygen gas can be continuously supplied.

Since the pressure changes in the interior of the adsorption columns 113 accompanying the generation of concentrated oxygen are very large, periodic pressure fluctuations occur in the pressure in the oxygen gas flow path downstream of the adsorption columns 113 in accordance with the switching of the adsorption columns 113. The concentrated oxygen gas stored in the concentrated oxygen buffer tank 115 is adjusted so that the pressure fluctuations are attenuated by the pressure control valve 116.

The oxygen flow rate of the concentrated oxygen gas, the pressure of which has been adjusted by the oxygen generation unit 11, is controlled by an oxygen flow rate adjustment unit 12 comprising a control valve 121 and a flow meter 122, and is supplied from the oxygen supply port 13 to the outside of the oxygen concentrator via a humidifier 101. Note that either of the control valve 121 and the flow meter 122 in the oxygen flow rate adjustment unit 12 may be provided upstream of the flow path, and the oxygen flow rate adjustment unit 12 may have other components. Further, in place of the flow meter 122 and the control valve 121, a method in which the flow rate is switched by a switching-type fixed orifice or a method in which a visible flow meter such as a rotor meter is used as the flow meter 122 and the flow rate is adjusted by manual operation using a manual flow adjustment valve instead of the control valve 121 may be adopted, or another flow adjustment method may be used. Further, the oxygen concentrator 1 can be configured without a humidifier 101.

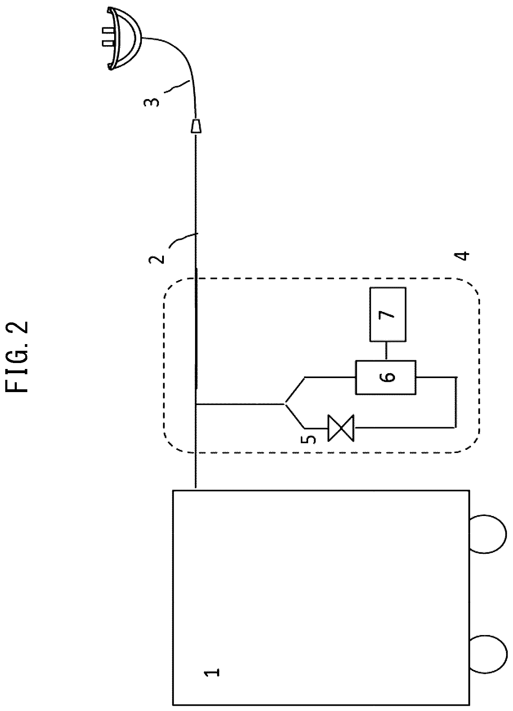

FIG. 2 schematically shows an example of the embodiment of the present technology and is an example of an aspect for acquiring patient respiratory information.

The oxygen generated by the oxygen concentrator 1 is supplied to the patient via an oxygen supply unit. The oxygen supply unit serves as an oxygen supply path, and includes an extension tube 2, a nasal cannula 3, and conduit between the humidifier 101 (or the oxygen flow rate adjustment unit 12 when the oxygen concentrator 1 does not include the humidifier 101) and the nasal cannula 3. The patient breathes constantly even during oxygen inhalation, and the pressure changes caused by the breathing of the patient propagate toward the nasal cannula 3, the extension tube 2, the conduit, and the oxygen concentrator 1.

In the present embodiment, in order to obtain the respiratory pressure of the patient, the respiratory information acquisition device 4 is connected in the conduit serving as the oxygen supply path. FIG. 2 shows the case in which the respiratory information acquisition device 4 is connected to an extension tube as an example. Note that "conduit" refers to the components including all of the conduit between the humidifier 101 (or the oxygen flow rate adjustment unit 12 when the oxygen concentrator 1 does not include the humidifier 101) and the nasal cannula 3, and the respiratory information acquisition device 4 may be connected in any position of the conduit. Accordingly, the respiratory information acquisition device 4 may be installed inside the oxygen concentrator 1, or may be installed outside the oxygen concentrator 1 separate from the oxygen concentrator 1.

As a result of the rigorous investigation conducted by the present inventors, it has been clarified that, even after attenuating the pressure fluctuations by the pressure control valve 116, pressure fluctuations applied to the respiratory information acquisition device 4 include pressure fluctuations accompanying the pressurization and depressurization when concentrated oxygen gas is generated. Since the amplitude of the pressure fluctuations generated during the production of the concentrated oxygen gas is larger than that of the respiration pressure, and the amplitude of the respiration pressure is also reduced due to the pressure loss caused by passing through the oxygen flow path, it is difficult to measure the respiration pressure wave form of the patient directly from the pressure measured by the respiratory information acquisition device 4

In the present embodiment, the respiratory information acquisition device 4 includes, for example, a pressure sensor, preferably a differential pressure sensor 6, as a pressure detection unit for detecting the pressure in the conduit, and a calculation unit 7 electrically connected to the pressure detection unit. The respiratory information acquisition device 4 may have a pressure smoothing unit composed of a volume connected to the differential pressure sensor 6 and an orifice 5 connecting the conduit and the volume. The reason for providing the pressure smoothing unit is as described below.

Since the respiratory pressure of the patient is generally approximately $\pm 10$ to 100 Pa, it is preferable to use a sensor having a range of approximately $\pm 100$ Pa for the differential pressure sensor 6 in order for the respiratory information acquisition device 4 to obtain the respiratory pressure. In a situation in which oxygen is supplied from the oxygen concentrator, a supply pressure is continuously generated by the oxygen supply, and, for example, a supply pressure due to oxygen supply of approximately 300 Pa is present even at 1 LPM (liter per minute: liter/min). When one end of the differential pressure sensor 6 is connected to the oxygen supply path while the other end of the differential pressure sensor 6 is released to the atmosphere, the measurement range of the differential pressure sensor 6 is exceeded. When the pressure after passing through the orifice 5 is applied to the other end of the differential pressure sensor 6, the pressure including the respiratory information of the patient can be acquired within the measurement range of the differential pressure sensor 6, which is more preferable.

Note that it is sufficient that the pressure applied to the other end of the differential pressure sensor 6 be adjusted to the extent that it does not exceed the measurement range of the differential pressure sensor 6, as described above, and the method therefor is not limited to the example of the present embodiment. As long as the measurement range of the differential pressure sensor is greater than the supply pressure provided by the oxygen supply and the resolution of the level at which the pressure fluctuations associated with the patient respiration can be detected, the other end of the differential pressure sensor 6 may be open to the atmosphere, and a pressure sensor which measures gauge pressure or absolute pressure may be used in place of the differential pressure sensor. Further, since the pressure changes accompanying respiration also appear as minute flow rate fluctuations of the concentrated oxygen gas flowing in the conduit, for example, a flow rate sensor may be used as the flow rate detection unit for detecting the gas flow rate in the conduit in place of the pressure sensor.

The calculation unit 7 is connected to the differential pressure sensor 6. The calculation unit 7 includes an input/output interface, a memory, and a CPU (central processing unit). The calculation unit 7 receives and records the pressure including the respiratory information of the patient detected by the differential pressure sensor 6, and acquires the respiratory information of the patient by performing a process which is described later.

Further, the calculation unit 7 may control the opening and closing of the first switching valve 112 and the second switching valve 114 of the oxygen concentrator 1. In this case, the calculation unit can obtain the period for switching between pressurization and depressurization in the adsorption columns 113

[Principle of Processing Performed in Embodiment]

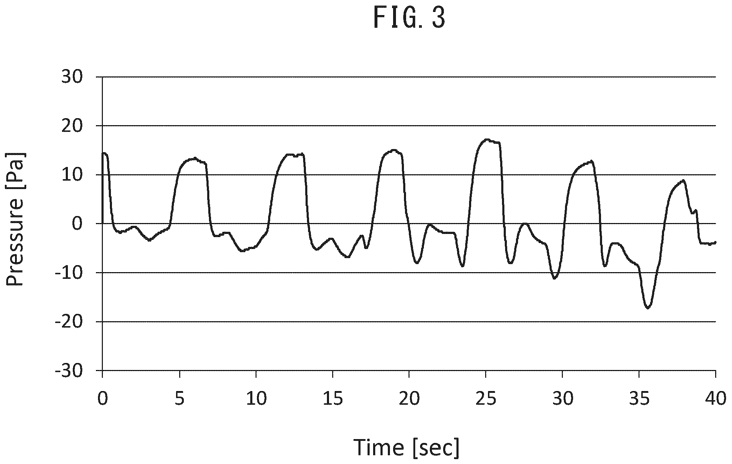

The principle of the processing performed in the present embodiment will be described using FIGS. 3 to 9. The principal of the processing will be described using the data acquired by the respiratory information acquisition device 4 when the respiratory pressure of the patient respiration model (expiratory pressure: 30 Pa, inspiratory pressure: -50 Pa, BPM: 20) is applied from the nasal cannula 3 as an example, using the configuration shown in FIG. 2.

FIGS. 3, 4, and 5 show data sets acquired by the respiratory information acquisition device 4 when a 10 m extension tube 2 was connected on the downstream-side of the respiratory information acquisition device 4 and under a 1 LPM continuous flow. "Continuous flow" is one concentrated oxygen gas supply method, and is a method of continuously supplying concentrated oxygen gas at a constant flow rate.

FIG. 3 shows the pressure data including the patient respiratory information and the PSA pressure data of the oxygen concentrator. The time period from 0 to approximately 15 seconds from the start of measurement is the time period in which only the PSA pressure of the oxygen concentrator is measured without applying the patient respiratory pressure.

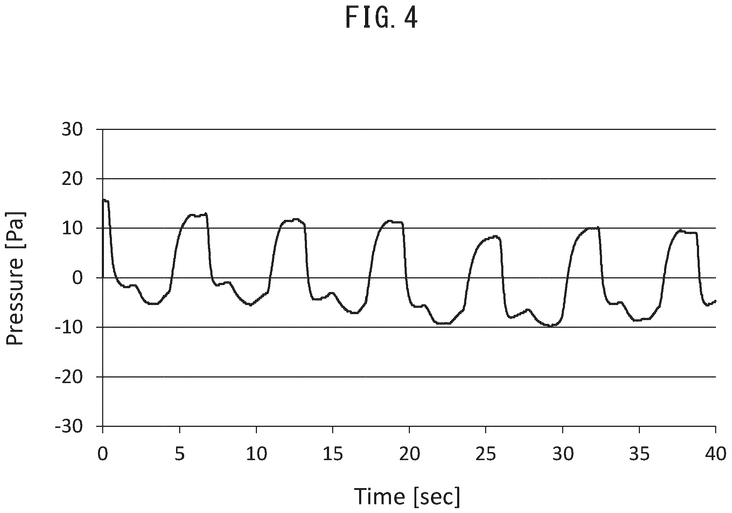

FIG. 4 shows the PSA pressure data of the oxygen concentrator acquired in advance and recorded in a storage medium. The PSA pressure of the oxygen concentrator has a periodic pressure change accompanying the adsorption columns interval of the PSA-type oxygen concentrator generated when oxygen is generated by the above-mentioned PSA-type oxygen concentrator. Thus, the interval of the PSA pressure waveform coincides with the adsorption columns switching interval T1 of the oxygen concentrator.

As shown in FIG. 4, the amplitude of the PSA pressure is approximately 20 Pa, and substantially the same fluctuation is repeated at interval T1. In FIG. 3, the patient respiration pressure is superimposed on the PSA pressure. However, since the PSA pressure is comparatively larger than the respiration pressure of the patient respiration model, the respiration pressure of the patient respiration model is drowned out by the PSA pressure and cannot be observed. Accordingly, the present inventors have conducted rigorous investigation and have devised a method for removing only the PSA pressure component by subtracting the component of FIG. 4 from the component of FIG. 3 by means of software to obtain the respiratory pressure of the patient respiration model.

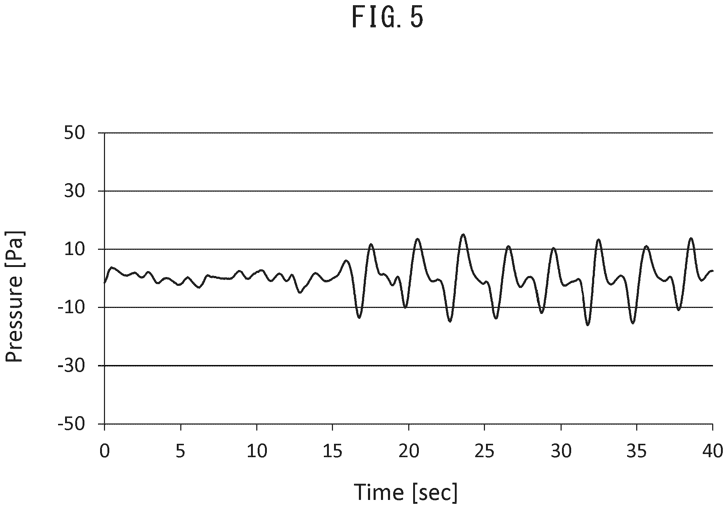

FIG. 5 shows the results of the subtraction processing of the components of FIG. 4 from the components of FIG. 3 by software. Note that the subtraction processing is a process of calculating the difference between both sets of data at an arbitrary time. In the subtraction processing, since the respiration pressure of the patient respiration model is not applied and the oxygen concentrator is in operation in the time from 0 to approximately 15 seconds, the characteristic waveforms are not seen. Conversely, periodic pressure fluctuations with an amplitude of approximately 20 Pa can be observed after approximately 15 seconds. Specifically, the respiratory pressure of the patient respiration model can be detected. By executing software subtraction processing, the patient respiratory information can be acquired even in a situation in which 1 LPM of oxygen is continuously inhaled from the oxygen concentrator through the nasal cannula and the 10 m extension tube.

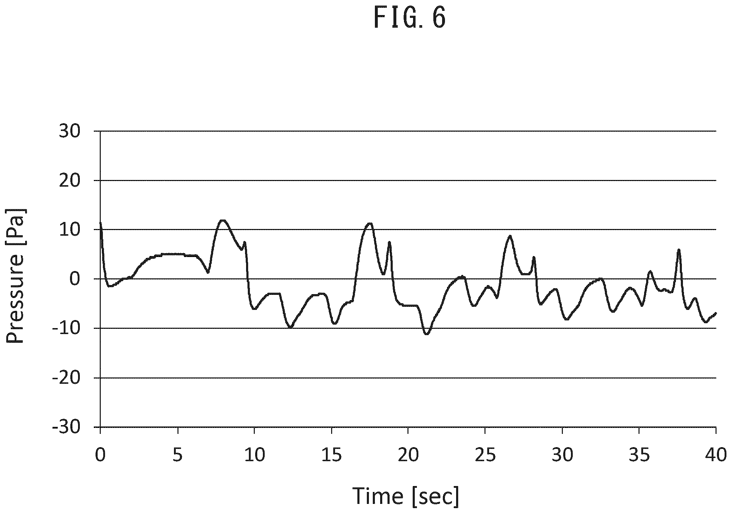

FIGS. 6, 7, and 8 show data sets acquired when the 10 m extension tube was connected on the downstream side of the respiratory information acquisition device and under a continuous-flow of 3 LPM.

FIG. 6 shows pressure data including the patient respiratory information and the oxygen concentration PSA pressure data, similarly to FIG. 3. The time period from 0 to approximately 10 seconds from the start of measurement is the time period during which only the PSA pressure of the oxygen concentrator is measured without the application of the patient respiration pressure.

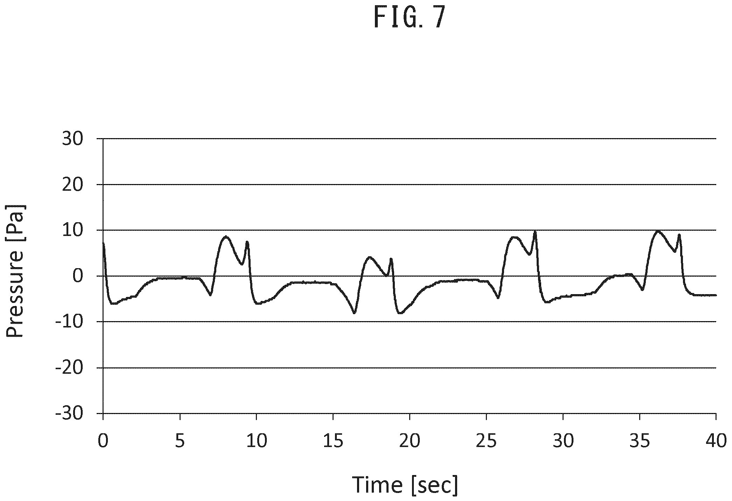

FIG. 7 shows the PSA pressure data of the oxygen concentrator previously acquired and recorded in the storage medium. The change in the shape of the waveform form compared with FIGS. 7 and 4 is due to the fact that the driving cycle for adsorbing and desorbing nitrogen with the adsorbent material when generating concentrated oxygen is different for each oxygen supply flow rate to be generated. However, the variation interval of the PSA pressure coincides with the adsorption columns switching interval, as in FIG. 4.

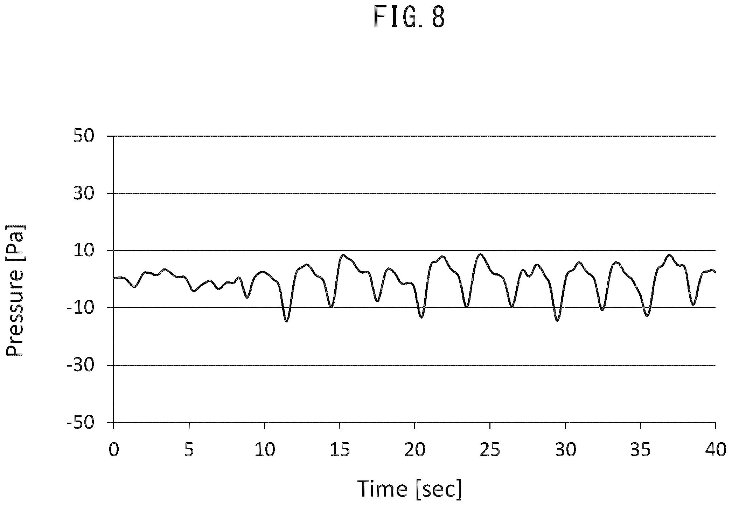

Similarly to FIG. 3, in FIG. 6, the respiratory pressure of the patient respiration model is superimposed on the PSA pressure, but the respiratory pressure of the patient respiration model is drowned out by the PSA pressure because the PSA pressure is comparatively larger than the respiratory pressure of the patient respiration model, even under the above conditions, and thus, cannot be observed. Similarly to FIG. 5, the results of the subtraction processing in a state in which the phases of both sets of data are combined is shown in FIG 8. Similarly to FIG. 5, a pressure fluctuation of approximately 20 Pa amplitude, which is observed periodically from approximately 10 seconds after the start of measurement in which the respiratory pressure is applied, can be observed. Specifically, the respiratory pressure of the patient respiration model can be detected. According to the method of performing subtraction processing with the phases of the respiration pressure and the PSA pressure matched, it can be seen that the patient respiratory information can be accurately acquired even under the conditions in which the 10 m extension tube is added or the supply oxygen flow rate is set to 3 LPM.

If the PSA pressure of the oxygen concentrator is known, it is possible to obtain respiratory information by subtraction processing. However, as a method of acquiring the PSA pressure used for the subtraction processing, in the method of recording the PSA pressure in the storage medium in advance, it is necessary to record the PSA pressures in advance under various operating conditions, and a large amount of storage capacity is required. Furthermore, it is impossible to respond to the case in which the PSA pressure changes due to changes of the surrounding environment or changes over time. As a result of rigorous investigation, the inventors have focused on the fact that a constant pattern of the PSA pressure is repeated with respect to the adsorption columns switching interval, and have discovered that the PSA pressure can be estimated by acquiring a variation history of the pressure and/or the flow rate in the conduit and extracting variations appearing in the same interval as the switching interval of the PSA operation therefrom.

Specifically, the inventors have discovered that by dividing the variation history of the pressure (or flow rate) in the conduit for each switching interval of the PSA operation, and adopting the average value of the variation waveforms of the plurality of intervals, variations other than the components synchronized with the switching interval of the PSA operation are attenuated, and the PSA pressure can be accurately estimated. It is possible to extract the respiratory information of the patient by utilizing the estimate of the PSA pressure for the above subtraction processing.

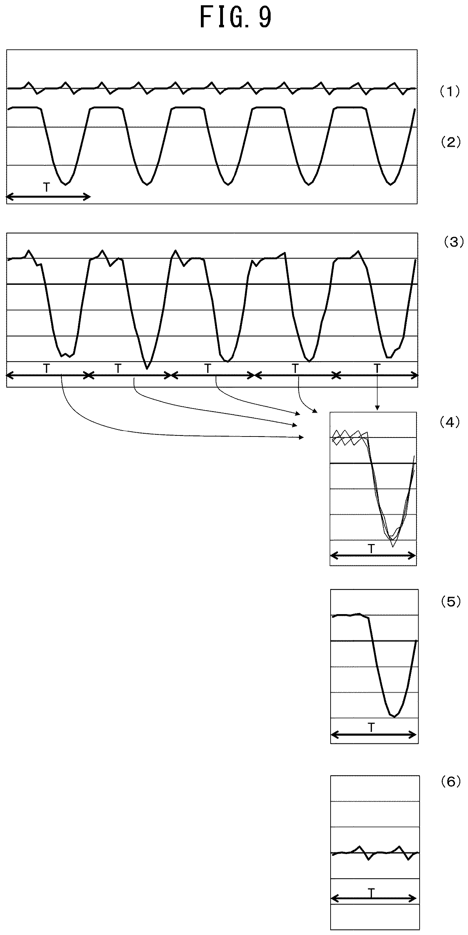

FIG. 9 is a conceptual diagram of the above arithmetic processing. (1), (2) are model waveforms of respiratory and PSA waveforms, respectively. T is the interval of the PSA waveforms, which is a known amount that matches the switching interval of the adsorption columns. (3) is a waveform obtained by adding (1) and (2), which corresponds to the pressure actually measured in the conduit. In (4), (3) is divided and superimposed for each interval T. Respiratory waveforms appear randomly in T-cropped waveforms unless the interval of respiration and the interval of PSA are perfectly matched. (5) is the waveform obtained by averaging the waveforms superimposed in (4). In this example, a simple moving average of five intervals is taken using T as a single interval. By performing averaging processing as shown in FIG. 9, the respiratory waveform component that is not consistent with the PSA interval is greatly attenuated, whereby it can be seen that the original PSA waveforms can be accurately reproduced. (6) is the trace obtained by subtracting (5) from the final T portion of the waveform in (3). Due to the averaging processing, the original respiration waveform (1) can be reproduced with suitable accuracy.

Note that the interval T may be selected by setting the time period from the start of the state in which one of the plurality of adsorption columnss is connected with the compressor until a state in which another adsorption columns is switched to a state in which it is connected with the compressor as T. Alternatively, the interval T may be selected by setting the time period from the start of the state in which one adsorption columns is connected with the compressor, and then after the state in which another adsorption columns communicates with the compressor, until a state in which the first adsorption columns is switched to a state in which the first adsorption columns communicates with the compressor again. In addition, an integral multiple of each time may be set as T.

Furthermore, though a moving average of five intervals is adopted in the above description when calculating the averaging process of (5), another averaging method can be used. Generally, the average value X(t) calculated in the step of (5) is represented by the following equation. Note that i represents an arbitrary positive integer.

[Math. 2]

$$X(t) = \frac{\sum_{i=0}^{n} a_i Y(t - iT)}{\sum_{i=0}^{n} a_i} \qquad \cdots (1)$$

In equation (1), X(t) is a value after the averaging process at time t, Y(t) is an actual measurement of the pressure at time t. $a_i$ is a weight in the weighted average, and any real number can be selected. n indicates the number of Ys averaged. For example, if n = 4 and $a_0$ to $a_4$ is 1, the calculation is the same as in FIG. 9. Further, if $a_i = e^{-bi}$ (b is any positive real number), and n = ∞, exponential smoothing averaging can be obtained. Since it is difficult to account for infinity in actual calculation, an asymptotic value is obtained by sequential computation such as $X(t) = (1^{-e-b})Y(t) + e^{-b}X(t - T)$. Further, by appropriately selecting n and $a_i$, faster converging averaging processing such as FIR-filtering can be performed.

Though equation (1) appears to be a simple numerical calculation equation for the time average of Y(t) at a glance, it is important that as the normal time average, a value sufficiently small with respect to the fluctuation interval of Y(t) be selected as T, whereas in the present embodiment, the adsorption/desorption interval of the PSA process is basically selected as T. Equation (1) is not simply for performing averaging processing of the measured values Y(t) in terms of time, but rather means that the waveform of Y(t) over interval T is used as one unit, and the averaging is performed with respect to the values that are traced back by the integral multiple times of T. It is possible to accurately estimate the PSA pressure having the characteristic of appearing periodically at intervals of T.

In the respiratory information acquisition device, a method of measuring pressure has been described, but in practice, a method of measuring a gas flow rate instead of pressure can also be used since the flow rate of the gas flowing through the conduit also changes with variations in pressure. It is also possible to use a method of measuring by combining or switching the pressure value and the gas flow rate value according to the operating conditions and environmental conditions of the device.

For example, the respiratory information acquisition device 4 infers a set flow rate corresponding to the flow rate measurement result, and can determine which of the adsorption columns switching intervals T for each set flow rate stored in advance in the memory, for example, should be used. This is useful when the respiratory information acquisition device 4 is detached from the oxygen concentrator 1 and is externally attached.

[Arithmetic Processing Performed in Embodiment 1]

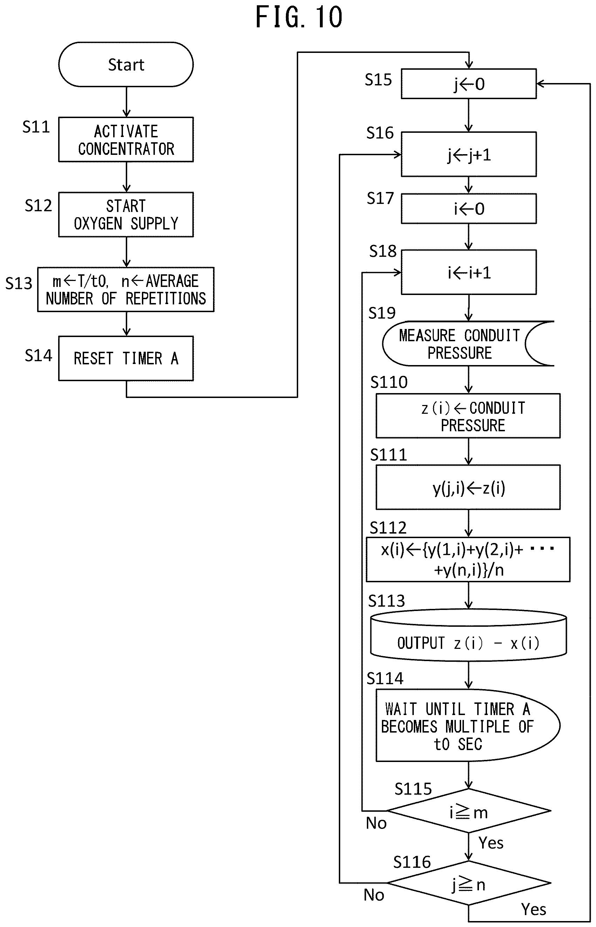

FIG. 10 is a flowchart illustrating an example of a specific processing method for acquiring respiratory information of a patient using the respiratory information acquisition device 4. This process is executed by the calculation unit 7.

First, in steps S11 and S12, the calculation unit 7 activates the oxygen concentrator and the oxygen supply is started. Thereafter, in step S13, in the calculation unit 7, the number of measurement repetitions m of pressure in the conduit during PSA interval T and the average number of repetitions n of the moving average are set as initial settings, and in step S14, the timer A for determining the measurement interval is reset, and the counting of the timer A starts. m is, for example, the ratio T/t0 of the adsorption columns switching interval T and the measurement interval t0 of pressure in the conduit, and t0 is set by selecting a value sufficiently smaller than T and the respiration interval. Since the number of measurement repetitions m must be an integer, t0 must be an integral fraction of T. For example, when t0 is set to 100 ms, T may be set to be an integral multiple of 100 ms. For example, when the adsorption columns switching interval T is adjustable in units of 100 ms, if t0 = 100ms, the number of measurement repetitions m can correspond to an arbitrary adsorption columns switching interval T. Note that the number of measurement repetitions m and the average number of the moving average n are set as initial values in the memory, but the initial values may be changed.

Steps after step S15 correspond to the main loop, and are performed at intervals of t0.

In step S15, the calculation unit 7 sets the counter j to 0, and adds 1 to j in step S16. The counter j is a counter for counting the average number of the moving averaging. The processes from step S16 to step S116 are repeated until j exceeds the number of repetitions n set in step S13 (step S116).

In step S17, the calculation unit 7 sets 0 to the counter i, and adds 1 to i in step S18. The counter i is a counter for counting the number of measurements of pressure in the conduit during the known PSA interval T. The processes from step S18 to step S115 are repeated until i exceeds the number of repetitions m set in step S13 (step S115).

In step S19, the calculation unit 7 receives the pressure signal data acquired by the respiratory information acquisition device 4 through the input interface, and stores the received pressure signal data as z(i) in the memory in step S110.

In step S110, the calculation unit 7, after storing the data of z(i) in the memory, copies Z(i) to another memory area in step S111, which is set as y(j, i).

In the step S112, the calculation unit 7 performs averaging processing on the data of y(1, i), y (2, i), ...y(n, i), and obtains an average value x(i). The method of the averaging processing may be {y(1, i) + y(2, i) + ... + y(n, i)} ÷ n as an addition average, or appropriate weighted averaging may be performed. By performing averaging with a sufficiently large n, the pressure components other than those which fluctuate in synchronism with the adsorption columns switching interval T are attenuated, and accordingly, it is possible to accurately estimate the PSA pressure. In the step S113, the calculation unit 7 outputs the difference z(i) - x(i) between the pressure measurement value and the average value x(i) as the respiratory information.

In step S114, the calculation unit 7 confirms the value of the timer A and waits until the value of the timer A becomes a multiple of t0. Thereafter, the calculation unit 7 proceeds to step S115 when the value of the timer A becomes a multiple of t0. The interval between data collection and data processing is adjusted to t0.

When i is m or more in step S115, the process proceeds to step S116, and when i is less than m, the process returns to step S18. If m = T / t0, m number of pressure signal data is acquired at t0 intervals during the interval T when i = m. In the present embodiment, by repeating step S18 to the step S115 until i = m, the calculation unit 7 collects m sets of data over the interval T and stores them in the memory. As a result, data is collected for a time period of one interval T.

In order to perform the averaging process, it is necessary to obtain a plurality of sets of data for the time period of the interval T. When i becomes m or more in step S115, the time T of one PSA interval has elapsed, and the respiration signal data of a subsequent interval T is acquired. Thus, by repeating step S16 to step S116 until step j = n, the calculation unit 7 can acquire the pressure signal data m times per interval T over the n times interval T. Specifically, it is determined that j is less than n in step S116, and after returning to step S16, in step S110, the calculation unit 7 updates the value of z(i) with the new pressure signal data at the time interval of t0, and this data is copied to the memory area of y(j, i) with respect to the value of j updated in step S16 (step S111). This is repeated n times and a total of m × n sets of data are acquired.

When the data processing of m × n is completed in step S16 to step S116, i.e., when j becomes n or more in S116, the process returns to step S15 and j is set to 0. The calculation unit 7 will again continuously update the data of z(i), and the copy destination of the data returns to y(1, i). Specifically, the calculation unit 7 maintains the values from y(2, i) to y(n, i) while updating the data of y(1, i) with new measurement data. The calculation unit 7 obtains a moving average value by performing the averaging processing again and updating x(i), and calculates the value of z(i) - x(i) therefrom, whereby the respiratory information can be continuously obtained.

According to the present embodiment, it is possible to separate the patient respiration component and the pressure fluctuation component associated with the PSA from the data obtained by measuring the patient respiration pressure during the operation of the oxygen concentrator to obtain the patient respiratory information.

[Arithmetic Processing Performed in Second Embodiment]

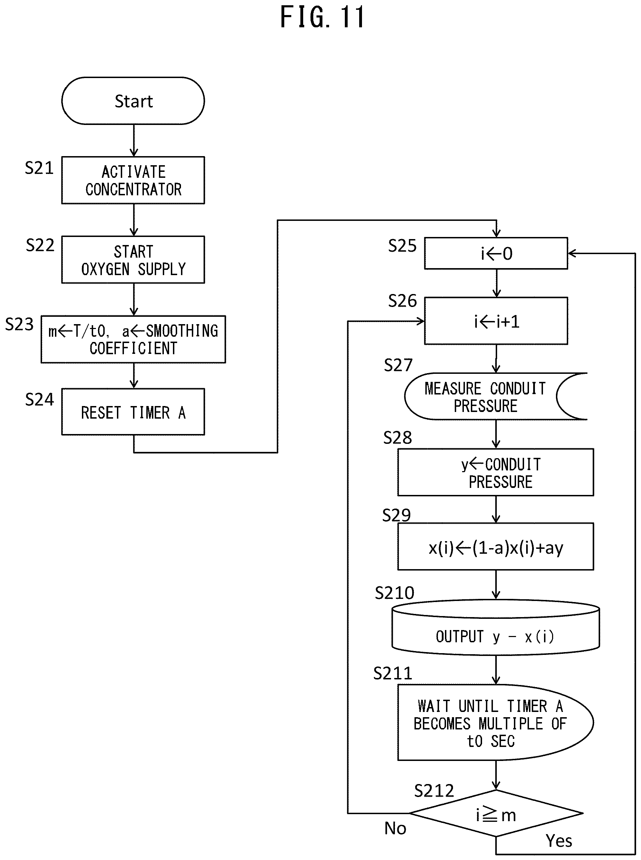

FIG. 11 is a flowchart illustrating another example of a processing method for acquiring respiratory information of a patient using the respiratory information acquisition device 4. This process is performed by the calculation unit 7 in the same manner as in the first embodiment. The operations of steps S21 and S22 are the same as those of steps S11 and S12 of the first embodiment. In step S23, the calculation unit 7 sets the value of the number of measurement repetitions m of the pressure in the conduit during the PSA interval T in the same manner as in S13 of the first embodiment, and sets the value of a smoothing coefficient a instead of the average number of repetitions n of the moving average in the first embodiment. In step S24, the calculation unit 7 resets the timer A and counting of the timer A starts.

Steps after step S25 constitute the main loop, and are executed at an interval of t0. Next, in S25, the calculation unit 7 sets a counter i for counting the number of measurement repetitions of pressure in the conduit during the known PSA interval T to 0 in the same manner as in the first embodiments, and in step S26, 1 is added to i.

The calculation unit 7 first collects the pressure signal from the respiratory information acquisition device 4 at interval t0 (step S27) in the same manner as in the first embodiment. The collected data is stored in y (step S28), and x(i) is updated by the equation x(i) ← (1 - a)x(i) + ay (a is a smoothing coefficient, and is a positive real number less than 1) (step S29). The updated x(i) is an estimate of PSA pressure. y - x(i) is the patient respiratory information (step S210). The value of i starts from 1, and 1 is added one by one after performing the process from collection of the above-mentioned pressure signal to extraction of respiratory information every t0 (step S26 to step S212), and returns to 1 again after m = T/t0 repetitions of processing (step S25).

The advantage of the present embodiment is that the number of memories and the number of operations required are less than those of the first embodiment, and the cost of the CPU and the memories can be saved. Conversely, a disadvantage is that if a large accidental pressure fluctuation occurs due to cannula breakage or the like, the effect remains for a long time, and a situation in which the estimation of the PSA pressure cannot be correctly performed continues for a long time.

With either means, the PSA pressure can be estimated accurately in a steady state, and the embodiment can be selected depending on the application and the allowable cost. In the present technology, it is important to estimate the PSA pressure by dividing the pressure fluctuation waveform in the conduit for each switching interval of the adsorption columns and averaging it over a plurality of intervals. The data acquisition method, the averaging method, and the memory usage method are not limited to the embodiments.

REFERENCE SIGNS LIST

**[0089]**

　　1 oxygen concentrator

# EP 3 858 408 B1

11 oxygen generation unit
12 oxygen flow rate adjustment unit
13 oxygen supply port
111 compressor
112 switching valve
113 adsorption columns
114 switching valve
115 concentrated oxygen buffer tank
116 pressure control valve
121 control valve
122 flowmeter
101 humidifier
2 extension tube
3 nasal cannula
4 respiratory information acquisition device
5 orifice
6 differential pressure sensor
7 calculation unit

## Claims

1. A respiratory information acquisition device (4) configured to acquire respiratory information of a patient used in an oxygen concentrator (1) comprising an oxygen generation unit (11) which has adsorption columns (113) having therein an adsorbent configured to adsorb at least nitrogen gas in air and configured to use a pressure swing adsorption method to concentrate oxygen in air by periodically repeating pressurization and depressurization in the adsorption columns, and an oxygen supply unit (2, 3) configured to supply concentrated oxygen gas which has been concentrated by the oxygen generation unit to the patient, which is a user, wherein

   the oxygen supply unit has at least a conduit for delivering the concentrated oxygen gas to the patient,
   the respiratory information acquisition device comprises a pressure detection unit (6) configured to detect pressure in the conduit and/or a flow rate detection unit configured to detect a gas flow rate in the conduit, as well as a calculation unit (7) configured to extract oxygen respiratory information from detected pressure data and/or flow rate data, and
   the calculation unit is configured to estimate a fluctuation component which does not depend on the respiration of the patient based on the detected pressure data and/or flow rate data and information related to an operation state of the oxygen concentrator obtained from the oxygen concentrator, and to extract respiratory information by removing the estimated fluctuation component from the detected pressure data and/or flow rate data.

2. The respiratory information acquisition device according to claim 1, wherein the calculation unit estimates a fluctuation value generated in accordance with operation of the oxygen generation unit among the detected pressure data and/or flow rate data as the fluctuation component.

3. The respiratory information acquisition device according to claim 2, wherein the calculation unit uses a fluctuation value of the pressure and/or the gas flow rate in the conduit associated with the pressurization and/or depressurization in the adsorption column as the fluctuation value.

4. The respiratory information acquisition device according to claim 3, wherein the calculation unit acquires switching period information related to a switching period at which the pressurization and depressurization in the adsorption columns is switched from the oxygen concentrator as the information related to the operation state of the oxygen concentrator, and estimates the fluctuation value based on the detected pressure data and/or flow rate data and the switching period information.

5. The respiratory information acquisition device according to claim 4, wherein the calculation unit estimates the fluctuation component of the pressure and/or gas flow rate fluctuates in the same cycle as the switching period included in the detected pressure data and/or flow rate data as the fluctuation value of the pressure and/or gas flow rate associated with the pressurization and/or depressurization in the adsorption columns.

**6.** The respiratory information acquisition device according to claim 5, wherein the oxygen generation unit switches between pressurization and depressurization of the adsorption columns at a constant interval T for at least a part of a total operation time, and the calculation unit extracts the respiratory information by calculating an averaging processed value X(t) using at least a part of the pressure or gas flow rate value Y(t) in the conduit at a certain time (t) and the values Y(t-T), Y(t-2T), ..., at the time retroactive by an integral multiple of T from t, among the detected pressure data and/or flow rate data, as the fluctuation value, and calculating the difference between Y(t) and X(t).

**7.** The respiratory information acquisition device according to claim 6, wherein the value X(t) is calculated by the formula represented by:

[Math 3]

$$X(t) = \frac{\sum_{i=0}^{n} a_i Y(t - iT)}{\sum_{i=0}^{n} a_i}$$

where $a_i$ is an arbitrary real number and n is an arbitrary natural number.

**8.** The respiratory information acquisition device according to claim 6 or 7, wherein exponential smoothing averaging, moving averaging, or weighted moving averaging is performed as the averaging processing.

**9.** The respiratory information acquisition device according to any one of claims 6 to 8, wherein the difference between an actual value of the pressure and/or gas flow rate in the conduit at t and a time average value of the pressure in the conduit and/or the flow rate in the conduit at t over a time equal to or greater than a respiration interval is used as the Y(t).

**10.** The respiratory information acquisition device according to any one of claims 6 to 8, wherein the pressure detection unit is a differential pressure sensor, the device further comprises a pressure smoothing unit, and a differential pressure between the pressure in the conduit at t and the pressure branched from the conduit and passed through the pressure smoothing unit is measured as Y(t).

**11.** The respiratory information acquisition device according to claim 10, wherein the pressure smoothing unit is constituted by a volume unit connected to the differential pressure sensor and an orifice for connection between the conduit and the volume unit.

**Patentansprüche**

**1.** Vorrichtung (4) zur Erfassung von Ateminformationen, die so konfiguriert ist, dass sie Ateminformationen eines Patienten erfasst, die in einem Sauerstoffkonzentrator (1) verwendet werden, umfassend eine Sauerstofferzeugungseinheit (11), die Adsorptionssäulen (113) mit einem darin befindlichen Adsorptionsmittel aufweist, das so konfiguriert ist, dass es zumindest Stickstoffgas in Luft adsorbiert, und das so konfiguriert ist, dass es ein Druckwechseladsorptionsverfahren verwendet, um Sauerstoff in Luft durch periodisches Wiederholen von Druckbeaufschlagung und Druckabsenkung in den Adsorptionssäulen zu konzentrieren, und eine Sauerstoffversorgungseinheit (2, 3), die dazu konfiguriert ist, dem Patienten, der ein Benutzer ist, konzentriertes Sauerstoffgas zuzuführen, das von der Sauerstofferzeugungseinheit konzentriert wurde, wobei

die Sauerstoffversorgungseinheit mindestens eine Leitung zum Abgeben des konzentrierten Sauerstoffgases an den Patienten aufweist,
die Vorrichtung zur Erfassung von Ateminformationen eine Druckerfassungseinheit (6), die dazu konfiguriert ist, Druck in der Leitung zu erfassen, und/oder eine Durchflusserfassungseinheit, die dazu konfiguriert ist, eine Gasströmungsrate in der Leitung zu erfassen, sowie eine Berechnungseinheit (7) umfasst, die dazu konfiguriert ist, aus erfassten Druckdaten und/oder Durchflussdaten Sauerstoffatmungsinformationen zu extrahieren, und die Berechnungseinheit dazu konfiguriert ist, eine Schwankungskomponente, die nicht von der Atmung des Patienten abhängt, auf Grundlage der erfassten Druckdaten und/oder Durchflussdaten und von Informationen bezüglich eines Betriebszustands des Sauerstoffkonzentrators, die vom Sauerstoffkonzentrator erhalten werden, zu schätzen, und Atmungsinformationen durch Entfernen der geschätzten Schwankungskomponente aus den erfassten Druckdaten und/oder Durchflussdaten zu extrahieren.

2. Vorrichtung zur Erfassung von Ateminformationen nach Anspruch 1, wobei die Berechnungseinheit einen entsprechend einem Betrieb der Sauerstofferzeugungseinheit erzeugten Schwankungswert aus den erfassten Druckdaten und/oder Durchflussdaten als die Schwankungskomponente schätzt.

3. Vorrichtung zur Erfassung von Ateminformationen nach Anspruch 2, wobei die Berechnungseinheit als den Schwankungswert einen Schwankungswert des Drucks und/oder der Gasströmungsrate in der Leitung verwendet, der mit der Druckbeaufschlagung und/oder Druckentlastung in der Adsorptionssäule verbunden ist.

4. Vorrichtung zur Erfassung von Ateminformationen nach Anspruch 3, wobei die Berechnungseinheit Umschaltperiodeninformationen bezüglich einer Umschaltperiode, bei der die Druckbeaufschlagung und Druckentlastung in den Adsorptionssäulen vom Sauerstoffkonzentrator umgeschaltet wird, als die Informationen bezüglich des Betriebszustands des Sauerstoffkonzentrators erfasst, und den Schwankungswert basierend auf den erfassten Druckdaten und/oder Durchflussdaten und den Schaltperiodeninformationen schätzt.

5. Vorrichtung zur Erfassung von Ateminformationen nach Anspruch 4, wobei die Berechnungseinheit die Schwankungskomponente des Drucks und/oder der Gasströmungsrate, die im gleichen Zyklus schwankt wie die in den erfassten Druckdaten und/oder Durchflussdaten eingeschlossene Umschaltperiode, als den Schwankungswert des Drucks und/oder der Gasströmungsrate schätzt, der mit der Druckbeaufschlagung und/oder Druckentlastung in den Adsorptionssäulen verbunden ist.

6. Vorrichtung zur Erfassung von Ateminformationen nach Anspruch 5, wobei die Sauerstofferzeugungseinheit für mindestens einen Teil einer Gesamtbetriebszeit in einem konstanten Intervall T zwischen Druckbeaufschlagung und Druckentlastung der Adsorptionssäulen umschaltet, und die Berechnungseinheit die Atmungsinformationen extrahiert, indem sie unter Verwendung von zumindest einem Teil des Druck- oder Gasdurchflusswerts Y(t) in der Leitung zu einem bestimmten Zeitpunkt (t) und der Werte Y(t-T), Y(t-2T), ... zu dem um ein ganzzahliges Vielfaches von T rückwirkenden Zeitpunkt von t unter den erfassten Druckdaten und/oder Durchflussdaten als Schwankungswert einen durchschnittlichen verarbeiteten Wert X(t) berechnet und die Differenz zwischen Y(t) und X(t) berechnet.

7. Vorrichtung zur Erfassung von Ateminformationen nach Anspruch 6, wobei der Wert X(t) mit der Formel berechnet wird, dargestellt durch:

[Mathematik 3]

$$X(t) = \frac{\sum_{i=0}^{n} a_i Y(t - iT)}{\sum_{i=0}^{n} a_i}$$

wobei $a_i$ eine beliebige reelle Zahl ist und n eine beliebige natürliche Zahl ist.

8. Vorrichtung zur Erfassung von Ateminformationen nach Anspruch 6 oder 7, wobei als Mittelwertbildung eine exponentielle Glättungsmittelwertbildung, eine gleitende Mittelwertbildung oder eine gewichtete gleitende Mittelwertbildung durchgeführt wird.

9. Vorrichtung zur Erfassung von Ateminformationen nach einem der Ansprüche 6 bis 8, wobei die Differenz zwischen einem tatsächlichen Wert des Drucks und/oder der Gasströmungsrate in der Leitung bei t und einem zeitlichen Mittelwert des Drucks in der Leitung und/oder der Strömungsrate in der Leitung bei t über eine Zeit, die gleich oder größer als ein Atmungsintervall ist, als Y(t) verwendet wird.

10. Vorrichtung zur Erfassung von Ateminformationen nach einem der Ansprüche 6 bis 8, wobei die Druckerfassungseinheit ein Differenzdrucksensor ist, die Vorrichtung weiter eine Druckglättungseinheit umfasst, und ein Differenzdruck zwischen dem Druck in der Leitung bei t und dem von der Leitung abgezweigten und durch die Druckglättungseinheit geleiteten Druck als Y(t) gemessen wird.

**11.** Vorrichtung zur Erfassung von Ateminformationen nach Anspruch 10, wobei die Druckglättungseinheit aus einer Volumeneinheit gebildet ist, die mit dem Differenzdrucksensor verbunden ist, und einer Öffnung zum Verbinden zwischen der Leitung und der Volumeneinheit.

**Revendications**

**1.** Dispositif d'acquisition d'informations respiratoires (4) configuré pour acquérir des informations respiratoires d'un patient utilisé dans un concentrateur d'oxygène (1) comprenant une unité de génération d'oxygène (11) qui comporte des colonnes d'adsorption (113) dans lesquelles un adsorbant configuré pour adsorber au moins de l'azote gazeux dans l'air et configuré pour utiliser un procédé d'adsorption modulée en pression pour concentrer l'oxygène dans l'air en répétant périodiquement la mise sous pression et la dépressurisation dans les colonnes d'adsorption, et une unité d'alimentation en oxygène (2, 3) configurée pour distribuer de l'oxygène gazeux concentré qui a été concentré par l'unité de génération d'oxygène au patient, qui est un utilisateur, dans lequel

l'unité d'alimentation en oxygène comporte au moins un conduit pour administrer l'oxygène gazeux concentré au patient,
le dispositif d'acquisition d'informations respiratoires comprend une unité de détection de pression (6) configurée pour détecter la pression dans le conduit et/ou une unité de détection de débit configurée pour détecter un débit de gaz dans le conduit, ainsi qu'une unité de calcul (7) configurée pour extraire des informations respiratoires d'oxygène à partir de données de pression et/ou données de débit détectées, et
l'unité de calcul est configurée pour estimer une composante de fluctuation qui ne dépend pas de la respiration du patient à partir des données de pression et/ou données de débit détectées et d'informations relatives à un état de fonctionnement du concentrateur d'oxygène obtenues à partir du concentrateur d'oxygène, et pour extraire des informations respiratoires en supprimant la composante de fluctuation estimée des données de pression et/ou données de débit détectées.

**2.** Dispositif d'acquisition d'informations respiratoires selon la revendication 1, dans lequel l'unité de calcul estime une valeur de fluctuation générée en fonction du fonctionnement de l'unité de génération d'oxygène parmi les données de pression et/ou données de débit détectées en tant que composante de fluctuation.

**3.** Dispositif d'acquisition d'informations respiratoires selon la revendication 2, dans lequel l'unité de calcul utilise comme valeur de fluctuation une valeur de fluctuation de la pression et/ou du débit de gaz dans le conduit associée à la mise sous pression et/ou à la dépressurisation dans la colonne d'adsorption en tant que valeur de fluctuation.

**4.** Dispositif d'acquisition d'informations respiratoires selon la revendication 3, dans lequel l'unité de calcul acquiert des informations de période de commutation relatives à une période de commutation selon laquelle la mise sous pression et la dépressurisation dans les colonnes d'adsorption sont commutées à partir du concentrateur d'oxygène en tant qu'informations relatives à l'état de fonctionnement du concentrateur d'oxygène, et estime la valeur de fluctuation sur la base des données de pression et/ou données de débit détectées et des informations de période de commutation.

**5.** Dispositif d'acquisition d'informations respiratoires selon la revendication 4, dans lequel l'unité de calcul estime la composante de fluctuation de la pression et/ou du débit de gaz qui fluctue dans le même cycle que la période de commutation incluse dans les données de pression et/ou données de débit détectées comme étant la valeur de fluctuation de la pression et/ou du débit de gaz associée à la mise sous pression et/ou à la dépressurisation dans les colonnes d'adsorption.

**6.** Dispositif d'acquisition d'informations respiratoires selon la revendication 5, dans lequel l'unité de génération d'oxygène commute entre la mise sous pression et la dépressurisation des colonnes d'adsorption à un intervalle constant T pendant au moins une partie d'un temps de fonctionnement total, et l'unité de calcul extrait les informations respiratoires en calculant une valeur traitée moyenne X(t) en utilisant au moins une partie de la valeur de pression ou de débit de gaz Y(t) dans le conduit à un certain temps (t) et les valeurs Y(t-T), Y(t-2T),..., au temps rétroactif par un multiple entier de T à partir de t, parmi les données de pression et/ou données de débit détectées, comme valeur de fluctuation, et en calculant la différence entre Y(t) et X(t).

**7.** Dispositif d'acquisition d'informations respiratoires selon la revendication 6, dans lequel la valeur X(t) est calculée par la formule représentée par :

[Math 3]

$$X(t) = \frac{\sum_{i=0}^{n} a_i Y(t - iT)}{\sum_{i=0}^{n} a_i}$$

où $a_i$ est un nombre réel arbitraire et n est un nombre naturel arbitraire.

8. Dispositif d'acquisition d'informations respiratoires selon la revendication 6 ou 7, dans lequel un calcul de moyenne de lissage exponentiel, un calcul de moyenne mobile ou un calcul de moyenne mobile pondérée est effectué en tant que traitement de calcul de moyenne.

9. Dispositif d'acquisition d'informations respiratoires selon l'une quelconque des revendications 6 à 8, dans lequel la différence entre une valeur réelle de la pression et/ou du débit de gaz dans le conduit à t et une valeur moyenne temporelle de la pression dans le conduit et/ou du débit dans le conduit à t sur un temps égal ou supérieur à un intervalle de respiration est utilisée comme étant Y(t).

10. Dispositif d'acquisition d'informations respiratoires selon l'une quelconque des revendications 6 à 8, dans lequel l'unité de détection de pression est un capteur de pression différentielle, le dispositif comprenant en outre une unité de lissage de pression, et une pression différentielle entre la pression dans le conduit à t et la pression dérivée du conduit et traversant l'unité de lissage de pression est mesurée comme Y(t).

11. Dispositif d'acquisition d'informations respiratoires selon la revendication 10, dans lequel l'unité de lissage de pression est constituée d'une unité de volume reliée au capteur de pression différentielle et d'un orifice de raccordement entre le conduit et l'unité de volume.

FIG. 1

FIG. 2

# FIG. 3

EP 3 858 408 B1

FIG. 4

EP 3 858 408 B1

FIG. 5

FIG. 6

FIG. 7

FIG. 8

EP 3 858 408 B1

# FIG. 9

(1)

(2)

(3)

(4)

(5)

(6)

# FIG. 10

Start

S11 ACTIVATE CONCENTRATOR

S12 START OXYGEN SUPPLY

S13 $m \leftarrow T/t0$, $n \leftarrow$ AVERAGE NUMBER OF REPETITIONS

S14 RESET TIMER A

S15 $j \leftarrow 0$

S16 $j \leftarrow j+1$

S17 $i \leftarrow 0$

S18 $i \leftarrow i+1$

S19 MEASURE CONDUIT PRESSURE

S110 $z(i) \leftarrow$ CONDUIT PRESSURE

S111 $y(j,i) \leftarrow z(i)$

S112 $x(i) \leftarrow \{y(1,i)+y(2,i)+ \cdots +y(n,i)\}/n$

S113 OUTPUT $z(i) - x(i)$

S114 WAIT UNTIL TIMER A BECOMES MULTIPLE OF t0 SEC

S115 $i \geqq m$
No
Yes

S116 $j \geqq n$
No
Yes

# FIG. 11

```
           ┌──────────────┐
           │    Start     │
           └──────────────┘
                  │
   S21     ┌──────────────┐
           │   ACTIVATE   │
           │ CONCENTRATOR │
           └──────────────┘
                  │
   S22     ┌──────────────┐
           │    START     │
           │ OXYGEN SUPPLY│
           └──────────────┘
                  │
   S23     ┌──────────────────────┐
           │ m←T/t0, a←SMOOTHING   │
           │      COEFFICIENT     │
           └──────────────────────┘
                  │
   S24     ┌──────────────┐
           │ RESET TIMER A│
           └──────────────┘
```

S25   i←0

S26   i←i+1

S27   MEASURE CONDUIT PRESSURE

S28   y←CONDUIT PRESSURE

S29   $x(i)←(1-a)x(i)+ay$

S210   OUTPUT $y - x(i)$

S211   WAIT UNTIL TIMER A BECOMES MULTIPLE OF t0 SEC

S212   $i \geqq m$     No     Yes

**EP 3 858 408 B1**

**REFERENCES CITED IN THE DESCRIPTION**

**Patent documents cited in the description**

- JP 6190045 A
- JP 2001286566 A
- JP 7096035 A
- JP 2015085191 A
- JP 2011518016 W
- WO 2014059405 A1